Europäisches Patentamt

European Patent Office

Office européen des brevets

(11) Publication number: **0 325 902**
**A1**

## (12) EUROPEAN PATENT APPLICATION

(21) Application number: **88850034.5**

(22) Date of filing: **29.01.88**

(51) Int. Cl.⁴: **A61F 5/453**

(43) Date of publication of application:
**02.08.89 Bulletin 89/31**

(84) Designated Contracting States:
**AT BE CH DE ES FR GB GR LI LU NL**

(71) Applicant: **AB MEDETT PRODUKTER**
**Box 8073**
**S-781 08 Borlänge(SE)**

(72) Inventor: **Von Matern, Christer**
**Medskogen 1**
**S-781 42 Borlänge(SE)**

(74) Representative: **Onn, Thorsten et al**
**AB STOCKHOLMS PATENTBYRA Box 3129**
**S-103 62 Stockholm(SE)**

(54) **A method and device for applying a urine drop collector onto a penis.**

(57) The invention relates to a method and a device for applying an elastic envelope, a urine drop collector (2), onto penis. The urine drop collector (2) is located in a substantially cylindrical applying sleeve (1) having a smooth outside (with a low frictional resistance) and at one (4) of its ends the urine drop collector is folded back over the applying end (5) of the sleeve. When the urine drop collector (2) is to be applied the sleeve (1) with the applying end (5) is moved upwards over penis and the other end thereof (3) is drawn out of the sleeve manually, the folded portion (4) of the urine drop collector being pulled over the edge (5) of the sleeve and sealed around penis.

EP 0 325 902 A1

## A Method and Device for Applying a Urine Drop Collector onto Penis

This invention relates to a method for applying an elastic envelope, a urine drop collector, onto penis and, further, a device for simplifying the application of an elastic envelope, a urine drop collector, onto penis.

Different methods are used for attachment of urine drop collectors onto penis. A usual method is using a fastening tape provided with an adhesive on both sides which is adapted on penis, after which the urine drop collector is rolled manually onto penis and will adhere to this. This method requires two moments, on one hand, and, on the other hand, it is often difficult for old persons to manage both of these.

US patent 4 475 910 discloses a urine drop collector which is rolled up and internally provided with a thin adhesive layer intended to hold the urine drop collector on penis when the latter is rolled on. Even with this it is often difficult to grasp the urine drop collector and roll it on. Moreover, the adhesive layer used is very thin, and therefore it will be difficult for the urine drop collector to remain in position and, furhter, this thin adhesive layer has difficulties in sealing if urine presses on from below.

SE lay-open prin 8004356-6 teaches a device for simplifying the application of a urine drop collector onto penis. That device is intended to widen the application end of the urine drop collector when this is to be attached to penis. After widening the application end of the urine drop collector the protective sheet must first be removed from an adhesive arranged internally in the urine drop collector. After this the device with the urine drop collector must be applied over penis and the urine drop collector be unrolled over the same. It is also difficult with this device to have the urine drop collector be attached around penis, especially for old persons and when penis is short or retracted as is often the case with old persons.

It is the object of this invention to provide an improved method for applying an elastic envelope, a urine drop collector, onto penis, and also to provide a device for simplifying the application of an elastic envelope onto penis.

The above-mentioned object has been achieved in that the invention has been given the characteristic features defined in the claims.

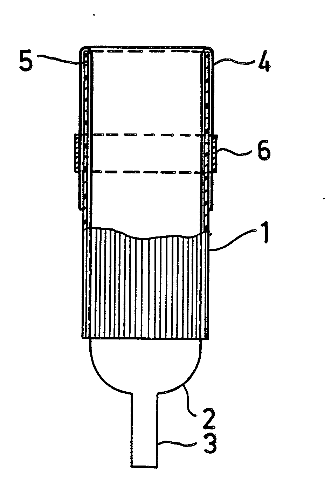

The invention is now described in greater detail in the form of a non-limiting illustrative example illustrated on the enclosed drawing where the only figure shows a device according to the invention partly in section.

The device of the invention comprises a substantially cylindrical sleeve 1 within which a conventional, condom-like urine drop collector 2 is inserted which is provided with a tube 3 at one of its ends for connection in known manner to a urine collecting bag or the like. The other end 4 of the urine drop collector 2 is drawn out through the application end of the sleeve 1 and folded back outside the sleeve so that the inside of the urine drop collector is turned outwards at the end 4. An adhesive 6 is applied on this outwardly turned part of the urine drop collector in a tape around the latter which adhesive tape can be rather thick. Before the use a protective paper not shown is applied covering the outside of the adhesive.

The substantially cylindrical sleeve is preferably made of a plastic material having a certain sliding ability to enable that the folded end 4 of the urine drop collector 2 can be easily pulled off. In order to simplify this the sleeve, moreover, can be externally provided with grooves or the like to avoid that the urine drop collector fits tightly against the outside of the sleeve. At the application end 5 the sleeve 1 may possibly externally also be somewhat tapered towards the application end 5.

When the sleeve 1 with the urine drop collector placed therein is delivered the tube 3 is not drawn out outside the end of the sleeve in the way shown in the figure but the tube and this end of the urine drop collector are then completely located within the sleeve 1.

When a urine drop collector is to be applied to penis by means of the device according to the invention its end with the tube is first drawn out of the sleeve and the protective sheet is removed from the adhesive 6. The sleeve with the urine drop collector is thereafter moved upwards over penis and pressed against the penis root by means of the application end 5. The tube portion on the urine drop collector is thereafter gripped and the latter is drawn outwards, the sleeve 1 being held fast against the penis root. The folded end 4 of the urine drop collector 2 will then be drawn over the application end 5 of the sleeve and the previously outwardly turned surface of this part of the urine drop collector will then be turned inwards towards penis so that the adhesive 6 is also turned around the edge of the application end 5 and will be in contact with and adhere to penis. When the urine drop collector is drawn out the tube end 3 of the urine drop collector 2 is sealed manually, a negative pressure being formed within the urine drop collector, with the result that the walls of the urine drop collector seal around penis. A prerequiste for the folded end 4 of the urine drop collector 2 being drawn over the application end 5 of the sleeve is that the sleeve 1 has a very low frictional resis-

tance to the urir
resistance on the
several ways, e.
talcing, oiling or c

By the device
it is easy and sa
onto penis so that
retracted. It is als
device.

## Claims

1. A metho
urine drop co
being inserted
thereof folded a
said sleeve (1
envelope (2) be
and pressed ag
envelope is gr
and drawn out
envelope is dra
characterized
envelope (2) s
tive pressure
and penis for
collector to ca

2. A dev
elastic envelo
penis, said ca
drical sleeve
inserted and
(4) back aro
characterized
provided with a
a tubular end
urine collecting
end (3) is form
applying the ur
to create a ne
drop collector a

3. A device
the folded por
across a major
(1).

4. The dev
in that a tace
inside of the en
of the envelope
on the folded-ca
consequently ic
(1).

5. The dev
acterized in th
material or arc
ability.

6. The device of any one of claims 2-5, **characterized** in that the sleeve (1) is externally provided with grooves in the longitudinal direction of the sleeve.

7. The device of any one of claims 2-6, **characterized** in that the sleeve (1), externally at the end (5) where the envelope (2) is folded, is tapered towards the end (5).

8. The device of any one of claims 2-7, **characterized** in that the outside of the sleeve (1) is coated with silicon, talced, oiled or provided with another sliding agent in order to have a very low frictional resistance against latex, rubber, a plastic material or another material of which the urine drop collector can be made.

European. Patent Office

**PARTIAL EUROPEAN SEARCH REPORT**
which under Rule 45 of the European Patent Convention shall be considered, for the purposes of subsequent proceedings, as the European search report

Application number

EP 88 85 0034

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US-A-4 540 409 (W. NYSTROM et al.) <br> * Figures 3-8; column 4, lines 7-9; column 5, lines 15-29; column 6, line 57 - column 7, line 19 * <br><br> -- | 2-5, 7,8 | A 61 F 5/453 |
| A | GB-A-2 120 102 (BARD LTD.) <br> * Abstract; figures 1,2 * <br><br> -- | 2 | |
| A | WO-A-87 01 582 (KAMSTRUP-LARSEN) <br> * Claim 1; figure 4 * <br><br> -- | | |
| A | WO-A-81 03 609 (BRENDLING) <br> * Figures 1,4; abstract * <br><br> --------- | | |

TECHNICAL FIELDS SEARCHED (Int. Cl.4)

A 61 F

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 2-8
Claims searched incompletely:
Claims not searched: 1
Reason for the limitation of the search:

See Article 52(1) of the European Patent Convention. Claim 1, although not a method for treatment of the human or animal body by therapy; is not susceptible of industrial application.

| Place of search | Date of completion of the search | Examiner |
|---|---|---|
| The Hague | 25-08-1988 | NEILL |

EPO Form 1505.1 03.82